# EUROPEAN PATENT SPECIFICATION

(11) **EP 3 075 373 B1**
(45) Date of publication and mention of the grant of the patent: **01.05.2024**
(21) Application number: 13898449.7
(22) Date of filing: 26.11.2013
(51) Int. Cl.: A61K 8/14, A61K 8/27, A61K 8/44, A61K 8/49, A61K 8/66, A61K 8/99, A61Q 19/00, A61Q 19/08

(54) **COSMETIC PRODUCT HAVING DNA REPAIR PROPERTIES**
KOSMETISCHES PRODUKT MIT DNA REPARIERENDE EIGENSCHAFTEN
PRODUIT COSMÉTIQUE AYANT DES PROPRIÉTÉS RÉPARATRICES DE L'ADN

(43) Date of publication of application: 05.10.2016
(73) Proprietor: Dermopartners, S.L., 46138 Rafelbunyol - Valencia (ES)
(72) Inventor: SERRANO SANMIGUEL, Gabriel, E-46138 Rafelbunyol - Valencia (ES); SERRANO NUÑEZ, Juan Manuel, E-46138 Rafelbunyol - Valencia (ES); TORRENS TOMAS, Ana, E-46138 Rafelbunyol - Valencia (ES)
(74) Representative: Urizar Anasagasti, Jesus Maria
(86) International application number: PCT/ES2013/070818
(87) International publication number: WO 2015/079074

(56) References cited:
- EP-A2- 0 707 844
- WO-A2-2011/162954
- DE-A1-102008 035 834
- ES-T3- 2 128 710
- ES-T3- 2 269 335
- ES-T3- 2 335 713
- US-A- 5 272 079
- US-A1- 2003 223 982
- US-A1- 2005 181 021
- US-A1- 2012 232 148

## Description

### OBJECT OF THE INVENTION

The proposed invention relates to a novel cosmetic product with DNA-repair properties for DNA which has suffered damage after exposure of the skin to UV radiation and which has an application in the sector of beauty, cosmetic and dermatology centers.

### BACKGROUND OF THE INVENTION

Different preparations for repairing DNA are known hitherto that include active ingredients of various types, but there are no known preparations that include phospholipid unilamellar liposomes with active ingredients such as: amino acids, zinc salts and DNA-repair enzymes.

The closest document known in the state of the art is the patent application

EP0707844, in which a product for repairing cellular DNA after exposure to UV radiation is described, where the active ingredients are vitamin and polypeptide complexes, tyrosine or the derivatives thereof, glycoproteins with copper metal ions, zinc or magnesium and DNA-repair enzymes.

The product described in this patent uses derivatives of tyrosine, vitamins in a protein and forskolin complex, unlike the product described in the present specification, in addition to incorporating a photolyase.

Furthermore, various active ingredients are encapsulated in the same liposome unlike the invention described here in which each active ingredient is separately encapsulated in the liposomes which provides greater stability to each one of the liposomes and thus to the final product.

The phosphatidylcholine used in the formulation of the liposome is a lecithin with > 94 % purity while the lecithin in the cited patent has a purity of > 25 %.

ES2269335T3 describes a cosmetic sunscreen skin agent incorporates a quantity of phospholipid-liposomes with a DNA repair enzyme within a substrate based on either alcoholic oil-in-water, a water in-oil emulsion, or an alcoholic hydrogel.

US2003223982A1 describes a method of using DNA repair enzymes in topical cosmetic or pharmaceutical compositions for the prevention of the ageing of human skin. In particular, the method prevents ageing effects caused by light. The method prevents the degradation of collagen within connective tissue, specifically within the dermis of the skin.

DE102008035834 describes a method for the production of liposome complexes with different active ingredients and products produced therewith. The liposome complexes comprise at least two liposomes with particle sizes in the range of 50-200 nm and with different active substances, the at least two liposomes being enclosed in a larger liposome which has a particle size of 250-600 nm, and the larger liposomes distributed in an aqueous gel with a viscosity in the range from 4000 to 20,000 mPa.s. It is produced by placing the at least two liposomes in water, adding a liposome former and a gel former to the mixture under special conditions and neutralizing them.

The patent application US2005181021 relates to protecting cells from damage and stimulating cell repair by administration of vitamin E phosphate encapsulated in phosphatidylcholine liposomes.

WO2011162954 describes a comprehensive, single agent cosmetic cream or lotion containing a high number of ingredients that target anti-aging in a defined manner. The cream or lotion contains a high number and variety of active substances that demonstrate excellent safety and efficacy in all of the various defined categories of skin aging, including but not limited to wrinkles, abnormal pigment or brown spots due to aging of the skin and an unexpectedly high efficacy in the reduction of redness and acne and rosacea blemishes.

There is no product known for repairing cellular DNA after exposure to UV radiation having the characteristics of the present invention.

### DESCRIPTION OF THE INVENTION

The product proposed by the invention is a cosmetic product with DNA-repair properties which comprises:
- an active ingredient that consists of a mixture of liposomes which have, in the interior thereof, repair enzymes such as Arabidopsis thaliana, plankton extract, bifida ferment lysate or micrococcus lysate
- a second active ingredient that consists of a mixture of liposomes which incorporate, in the interior thereof, amino acids such as arginine, glycine, proline, phenylalanine, cysteine, serine, tyrosine, glutamine or methionine
- a third active ingredient that is a zinc salt such as zinc chloride, zinc gluconate or zinc chlorohydrate, which is also found encapsulated in liposomes;
wherein each active ingredient is separately encapsulated in the liposomes.

In one embodiment of the invention, the liposomes encapsulate each one of the amino acids, the repair enzymes or the zinc salt separately.

Due to the metabolic pathways thereof, we can justify incorporating the following amino acids in the invention, in spite of them not being in the formula:
Serine: it is a cysteine and glycine precursor.
Tyrosine: the precursor thereof is phenylalanine. It is obtained by hydroxylating phenylalanine.
Methionine: intermediate product of cysteine synthesis. Cysteine obtains the atom of methionine sulfur.
Glutamine: it is synthetized by way of the chemical reaction between glutamine and the ammonium ion and glutamine can be converted into glutamate and proline is formed from the pentacarbonated chain of glutamic acid.

The exposure of the skin to UV radiation from the sun causes damage to the DNA of the skin cells. This damage can be measured, amongst other ways, by the thymine or cytosine dimers which are produced in the chain of cellular DNA.

### PREFERRED EMBODIMENT OF THE INVENTION

As a preferred embodiment, a liposome cosmetic product is proposed which presents different cosmetic forms such as a solution, serum, emulsion, suspension etc. in which the different components can be found in one of the cases in the following proportions by weight of active ingredient and liposomes.

| INCI COMPONENT | % total | % of liposomes |
|---|---|---|
| WATER | 70-80 | |
| GLYCERIN | 4-5 | |
| LECITHIN | 4-5 | |
| ALCOHOL | 3-4 | |
| HYDROGENATED CASTOR OIL PEG 40 | 2-3 | |
| CYCLOPENTASILOXANE | 2-3 | |
| POLYSORBATE 20 | 1 | |
| PHENOXYETHANOL | <1 | |
| SODIUM COLLATE | <0.5 | |
| CITRONELLYL METHYLCROTONATE | <0.5 | |
| PERFUME | <0.5 | |
| TOCOPHERYL ACETATE | <0.5 | |
| ARGININE | <0.5 | ARGININE 6-7 % |
| DIMETHICONE | <0.5 | |
| ETHYLHEXYLGLYCERIN | <0.5 | |
| POLYSILICONE-11 | <0.5 | |
| GLYCINE | <0.5 | GLYCINE 5-7 % |
| PROLINE | <0.5 | PROLINE 5-7 % |
| BUTYLENE GLYCOL | <0.5 | |
| LACTIC ACID | <0.5 | |
| EDTA DISODIUM | <0.1 | |
| SODIUM CHLORIDE | <0.1 | |
| BIFIDA FERMENT LYSATE | <0.1 | BIFIDA ENZYMES 7-8 % |
| CAPRYLIC/CAPRIC TRIGLYCERIDE | <0.1 | |
| PHENYLALANINE | <0.1 | PHENYLALANINE 5-7 % |
| CYSTEINE | <0.1 | CYSTEINE 5-7 % |
| ARABIDOPSIS THALIANA EXTRACT | <0.1 | ARABIDOPSIS 10-12 % |
| MICROCOCCUS LYSATE | <0.1 | MICROCOCCUS 10-12 % |
| SODIUM HYDROXIDE | <0.1 | |
| PLANKTON EXTRACT | <0.1 | PLANKTON 6-7 % |
| DECYL GLUCOSIDE | <0.1 | |
| ZINC CHLORIDE | <0.1 | ZINC 10-12 % |
| TEPRENONE | <0.1 | TEPRENONE 7-8 % |
| HYDROCHLORIC ACID | <0.1 | |
| CAPRYLYL GLYCOL | <0.1 | |
| HEXYLENE GLYCOL | <0.1 | |
| | 100,0000 | |

### DESCRIPTION OF THE DRAWINGS

In order to complement the description and with the aim of aiding a better understanding of the characteristics of the invention, a series of figures accompany the present specification as an integral part of the same.

One part of the figures included in this specification corresponds to the tests carried out on fish embryos of the medaka species with these types of products and which demonstrate the DNA repair capacity to us.

In the same way, we will see how the action mechanism for said DNA repair is the activation of the expression of proteins P53 and P21 which we will measure by quantitative PCR (qPCR).

In these figures, the following is depicted in an illustrative manner:

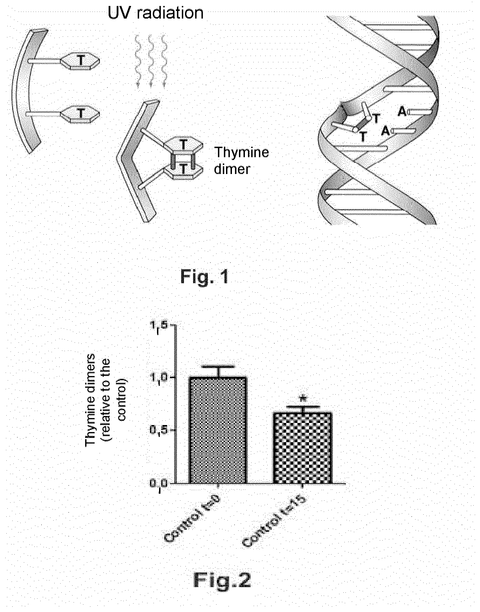

Figure 1 shows a diagram of the effect of UV radiation on the DNA chains and the formation thymine dimers between two bases of the same DNA helix.
Figure 2 presents a diagram of the damage caused to the DNA with respect to the control at time 0 and at 15 minutes. This test is carried out with UNTREATED samples, where the damage to the DNA (time = 0) increases, but the cell uses its own endogenic mechanisms to repair this damage and at 15' the TD levels are similar to the control.

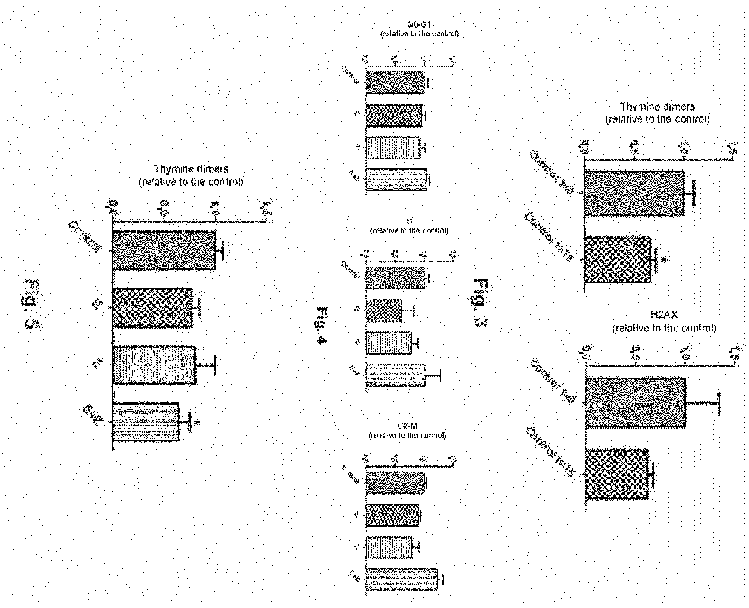

Figure 3 shows the variation of the expression of histone H2Ax and the thymine dimer levels with respect to the control at time 0 and at 15 minutes. Although we know that the phosphorylated H2Ax acts as a decoy for the enzymatic compounds for repairing DNA. The results at t = 0 and t 15' are not statistically significant.
Figure 4 depicts the variation of the cells in the phases of the cellular cycle G0-G1, G2-M and S at time 0 and at 15 minutes. The treatments DO NOT induce statistically significant changes in the cell distribution in the phases of the cellular cycle which can affect the results.
Figure 5 shows the TD levels, in the control case, it includes the base cream without the active ingredients, E: includes base cream with repair enzymes, Z: base cream with amino acids and Zn salt and E+Z: base cream with repair enzymes + amino acids + Zn salt. The combination of E+Z reduces the presence of TD by 36 % with respect to the control.

### CLINICAL TRIALS

In order to determine the efficacy of the product proposed by the invention trials are carried out in which three cosmetic preparations E, Z and E+Z are used. The trial is carried out *in vitro* on 4-day-old fish embryos in the eleutheroembryo stage.

Of the three products used in the study, product E contains a mixture of liposomes with repair enzymes (*Arabidopsis thaliana,* plankton extract, bifida ferment lysate, micrococcus lysate), product Z contains a mixture of liposomes with amino acids (arginine, glycine, proline, phenylalanine, cysteine) and zinc chloride and product E+Z contains the mixture of liposomes with all the repair enzymes cited in product E, plus the AA and Zn from product Z.

Firstly, the possible toxicity of the samples and the treatment time to be used in the trials were analyzed.

Control samples are prepared without irradiation, irradiated without treatment and irradiated and treated after the irradiation. The products are applied to the embryos before and after the irradiation in order to detect the possible repair of the DNA after inducing the damage.

To this end, an analysis of the presence of thymine dimers is carried out since UV radiation induces the formation of pyrimidine dimers in the DNA which can be detected by specific antibodies for these dimers (either thymine or cytosine). Using a specific antibody, we will quantify the quantity of thymine dimers induced by the UV radiation (see Fig. 1).

The samples are fixed and processed for the analysis thereof by flow cytometry which is carried out in the following manner:
The embryos are broken up with an enzymatic treatment with trypsin to separate the cells without damaging them. They are fixed with paraformaldehyde and are stained with the antibody for thymine dimers (TD) and for the compound to stain DNA (Hoescht) and calculate the quantity thereof. Based on the quantity of DNA, the phases of the cellular cycle in which the cells are located are determined.

After treating the samples with the creams, they are exposed to UV radiation and the cellular damage is analyzed based on the presence of thymine dimers induced by UV, the activation of the histone H2Ax and alterations in the cellular cycle.

After exposure to UV radiation, the damage to the DNA increases (time = 0) with respect to the untreated samples (C). The cell uses its own mechanisms for repairing this damage (34 %) and at 15 min (t = 15), the TD levels are similar to the control (see Fig. 2).

After exposure to the UV radiation, the expression of H2AX (time = 0) and at t = 15 increases, it is observed that the H2AX levels reduce similar to how the TD levels do so at t = 15, indicating a correlation between the expression of H2AX and the presence of TD. However, due to the high variability detected in the control, the reduction is not statistically significant (see Fig. 3).

The G0-G1 phase is the rest stage of the cell or preparation to enter into division. The G2-M phase is the separation of chromatids and cellular division (mitosis). The S phase is the synthesis of DNA. E+Z seems to slightly increase the G2-M phase, but the difference is not significant (see Fig. 4).

The combination of E+Z reduces the presence of thymine dimers by 36 ± 14 % (22-50 %) with respect to the control.

The control cream (empty) is the same for E, Z, and E+Z, but without these components. The samples are treated with the creams, irradiated and are analyzed, taking as a reference (C) the quantity of TD detected in the sample treated with the "empty" cream (see Fig. 5).

Lastly, the trial carried out to verify the activation of the action mechanisms which causes said repair should be pointed out. We select 2 proteins, P53 and P21, both are involved in this action mechanism and are activated by UV radiation.

The experiment is carried out twice, using 10 embryos, with a control group, without treatment with cream, of which half are treated with UV radiation for 15' and the other half are not. And the treated group where the cream is applied for 1 h.

After the qPCR analysis, the results are: after irradiation with UV light, the embryos treated with E+Z increase the genetic expression of P53 by 46 %. In the same way, the treatment with E+Z increases the expression of P21 at 15' of irradiation by 130 %.

## Claims

1. A cosmetic product with DNA-repair properties which comprises:
- an active ingredient that consists of a mixture of liposomes which have, in the interior thereof, repair enzymes such as Arabidopsis thaliana, plankton extract, bifida ferment lysate or micrococcus lysate
- a second active ingredient that consists of a mixture of liposomes which incorporate, in the interior thereof, amino acids such as arginine, glycine, proline, phenylalanine, cysteine, serine, tyrosine, glutamine or methionine
- a third active ingredient that is a zinc salt such as zinc chloride, zinc gluconate or zinc chlorohydrate, which is also found encapsulated in liposomes;
wherein each active ingredient is separately encapsulated in the liposomes.

2. The cosmetic product with DNA-repair properties according to claim 1, **characterized in that** the liposomes encapsulate each one of the amino acids, the repair enzymes or the zinc salt separately.

3. The cosmetic product with DNA-repair properties according to claim 1, **characterized in that** the phosphatidylcholine used in the formulation of the liposomes is a lecithin with a purity greater than 94 %.

## Patentansprüche

1. Ein kosmetisches produkt mit DNA-peparatureigenschaften, das folgendes umfasst:
- ein Wirkstoff, der aus einer Mischung von Liposomen besteht, die in ihrem Inneren Reparaturenzyme wie Arabidopsis thaliana, Planktonextrakt, Bifida-Ferment-Lysat oder Mikrokokken-Lysat enthalten
- einen zweiten Wirkstoff, der aus einer Mischung von Liposomen besteht, die in ihrem Inneren Aminosäuren wie Arginin, Glycin, Prolin, Phenylalanin, Cystein, Serin, Tyrosin, Glutamin oder Methionin enthalten
- ein dritter Wirkstoff, bei dem es sich um ein Zinksalz wie Zinkchlorid, Zinkgluconat oder Zinkchlorhydrat handelt, das auch in Liposomen eingekapselt vorkommt;
wobei jeder Wirkstoff separat in den Liposomen eingekapselt ist.

2. Kosmetisches produkt mit DNA-reparatureigenschaften nach anspruch 1, **dadurch gekennzeichnet, dass** die Liposomen jeweils eine der Aminosäuren, die Reparaturenzyme oder das Zinksalz separat verkapseln.

3. Kosmetisches produkt mit DNA-reparatureigenschaften nach anspruch 1, **dadurch gekennzeichnet, dass** das bei der Formulierung der Liposomen verwendete Phosphatidylcholin ein Lecithin mit einer Reinheit von mehr als 94 % ist.

## Revendications

1. Un produit cosmétique aux propriétés réparatrices de l'ADN qui comprend :
- un principe actif constitué d'un mélange de liposomes qui possèdent, à l'intérieur de ceux-ci, des enzymes réparatrices telles que Arabidopsis thaliana, extrait de plancton, lysat de ferment bifida ou lysat de micrococcus
- un deuxième principe actif constitué d'un mélange de liposomes qui incorporent à l'intérieur de ceux-ci des acides aminés tels que l'arginine, la glycine, la proline, la phénylalanine, la cystéine, la sérine, la tyrosine, la glutamine ou la méthionine
- un troisième principe actif qui est un sel de zinc tel que le chlorure de zinc, le gluconate de zinc ou le chlorohydrate de zinc, que l'on retrouve également encapsulé dans des liposomes ;
dans lequel chaque ingrédient actif est encapsulé séparément dans les liposomes.

2. Produit cosmétique aux propriétés réparatrices d'ADN selon la revendication 1, **caractérisé en ce que** les liposomes encapsulent séparément chacun des acides aminés, les enzymes de réparation ou le sel de zinc.

3. Produit cosmétique aux propriétés réparatrices d'ADN selon la revendication 1, **caractérisé en ce que** la phosphatidylcholine utilisée dans la formulation des liposomes est une lécithine de pureté supérieure à 94 %.
